# EUROPEAN PATENT SPECIFICATION

(11) **EP 0 431 933 B1**
(45) Date of publication and mention of the grant of the patent: **01.12.1993**
(21) Application number: 90313239.7
(22) Date of filing: 06.12.1990
(51) Int. Cl.: A61K 37/02, A61K 31/545, A61K 31/43

(54) **Formulated medicines for enhancing the efficacy of betalactam antibiotics in prophylaxis and treatment against infectious disease due to pathogenic bacteria**
Arzneimittelformulierungen zum Erhöhen der Wirksamkeit von Betalaktamantibiotika in Prophylaxe und Behandlung von infektiöser Krankheit infolge pathogener Bakterien
Médicaments formulés pour augmenter l'efficacité d'antibiotiques de bêtalactame en prophylaxie et traitement de maladie infectieuse par suite de bactéries pathogènes

(30) Priority: 08.12.1989 JP 319463/89
(43) Date of publication of application: 12.06.1991
(73) Proprietor: IMMUNO JAPAN INC., Tokyo 167 (JP)
(72) Inventor: Goto, Sachiko, Itikawa, Chiba 271 (JP)
(74) Representative: Arthur, Bryan Edward

(56) References cited:
- DE-A- 3 525 902
- ANTIMICROBIAL AGENTS AND CHEMOTHERAPY, vol. 32, no. 11, November 1988; MOCHIZUKI et al., pp. 1648-1654#

## Description

The present invention relates to formulated medicines for enhancement of the efficacy of beta-lactam antibiotics, both to prevent and treat infectious diseases caused by pathogenic bacteria. The formulated medicine of the present invention contains bovine milk lactoferrin and shows remarkable synergistic effect on the efficacy of beta-lactam antibiotics, thus more effectively preventing and treating infectious diseases of mammals, including human beings. The present invention also relates to a method for the prevention and treatment of infectious disease.

Lactoferrin is the major glycoprotein present in the granules of mature neutrophils, and is deemed to be one of the body's self-defensive factors, being locally released where pathogenic bacteria infect. Lactoferrin also exists in such diverse secretions as milk, tears, saliva, and digestive juices, and is thought to be one of the factors which prevent mammals from being infected by bacterial pathogens. The function of lactoferrin is considered similar to that of lysozymes and secretory immunoglobulins. The most important role of lactoferrin is thought to be the protection of mucous membranes that occupy huge areas on the body and which are always threatened by invasion of bacterial pathogens. Therefore, lactoferrin is regarded as a nonspecific barrier against pathogens. It probably cooperates with other defensive factors such as lysozymes and immunoglobulins.

In a healthy human adult male, 5 grams of lactoferrin is produced daily on the basis of the turnover rate of neutrophils. However, as it is well known, when acute inflammation caused by bacterial infection occurs, production of lactoferrin increases by approximately 6 fold to 30 g per day. For this reason lactoferrin is considered to play a very important role in host defense and its importance is comparable to other self-defensive factors such as immunoglobulins and lysozymes.

In mammals (including human beings), milk, especially colostrum, contains a large amount of lactoferrin. The reason why colostrum contains a large amount of lactoferrin is that it seems to protect newborn infants from pathogens since a fetus grows under aseptic conditions and never encounters bacterial pathogens before birth. Therefore, they are highly susceptible to bacterial pathogens because of the immature properties of their immune system in which such self-defensive factors as immunoglobulins and immunocytes with cellular immunity functions are produced. Their intestines are free from bacteria at birth and the normal intestinal microflora is not formed at that time. The role of lactoferrin in milk, especially in colostrum, in cooperation with other protective factors, is to cover the surface of the intestinal mucosa, thus protecting the intestine from infection by pathogens and to allow the newborn to form normal intestinal microflora. However, it is not clear how and where the lactoferrin acts in the body. In spite of a number of studies regarding lactoferrin, the answers to these questions have not been obtained yet. Moreover, no one can say what kind of response occurs when exogenous lactoferrins, derived from other animal species, are either given orally or parenterally.

Bovine milk lactoferrin, one of the biologically active proteins, is a minor component extracted from whey fraction, and whey, especially cheese whey, is one valuable natural resource which has not been fully utilized yet. For example, most of the whey is discarded during the process of cheese manufacturing. Today, owing to development of new technology, e.g., large scale separation and purification techniques using filter membranes and ion exchange gel column chromatography, such minor but valuable and biologically active proteins as lactoferrins can be efficiently extracted and isolated in native form from whey (which contains numerous minor ingredients). Therefore, relatively pure lactoferrin (approximately 85% pure) is now available at a reasonable cost owing to innovative technology, but this development is worthless without development of a potential usefulness for lactoferrin.

Two reasons are considered why the biomedical use of lactoferrin has been hindered. The first reason is that there is a possibility that the parenteral administration of macro-molecules derived from other animal species will result in severe anaphylactic shock when repeatedly administered; bovine lactoferrin is non-self for humans. The second reason originates from the digestibility of lactoferrin in the gastrointestinal tract. If lactoferrin is taken orally, it was thought to be digested by proteinase in the digestive tract and to be absorbed as inactive peptides. However, recent immunological studies indicate that intact foreign antigenic determinants are occasionally absorbed through the gut where suppressor T-cells recognize the non-self antigens, thus inhibiting the immunological response against it. That is to say, when intact proteins derived from other species are absorbed in the body without changing the complete structure, it is considered that a kind of immuno-tolerance is induced. Therefore, as far as the oral route is concerned, bovine lactoferrin never causes anaphylactic shock in humans. Moreover, lactoferrin molecules are highly resistant to proteinases and considerable amounts are excreted in intact form in feces.

According to previous studies, lactoferrin is known as an antibacterial protein present in the granules of neutrophils. However, the present invention has revealed that lactoferrin shows virtually no antibacterial activity against bacterial pathogens. The measurement of the minimum inhibitory concentrations against various strains of pathogens by the serial dilution method on agar plate is presented in Table 1. The minimum inhibitory concentrations of bovine lactoferrin were 1,000-10,000 times higher than those of beta-lactam antibiotics, that is, it could be said that lactoferrin actually has no antibacterial activity.

One object of the present invention was to develop efficient drug formulations in order to prevent and treat infectious diseases caused by pathogenic bacteria in mammals, including human beings. Another object was to develop a method for the prevention and treatment of infectious diseases and to enhance the efficacy of beta-lactam antibiotics.

We have found that lactoferrin, especially bovine milk lactoferrin, enhances the activity of beta-lactam antibiotics in vivo against experimental infections in mice. The effect is marked with a weight of lactoferrin : antibiotic of 1 : 1 to 1 : 10. When these were orally given simultaneously to mice, immediately or 1 hour after injection of Klebsiella pneumoniae, antibacterial efficacy was 2-10 times greater than with antibiotics alone.

The characteristics of this synergistic effect of bovine lactoferrin with beta-lactam antibiotics are as follows: (1) the combination shows this effect with both penicillin and cephalosporin families of beta-lactam antibiotics, (2) the synergy is observed not only in mice but also in human and other animal species. Co-administration of lactoferrin greatly reduces the amounts of the antibiotics required, that is, one-half to one-tenth of the antibiotics are sufficient for treating the infected animals (including humans). Oral administration is an effective route, and the dose of lactoferrin required for this synergistic effect is 0.5-100 mg/kg and the optimum dose may be 1-10 mg/kg.

Lactoferrin isolated from either milk or neutrophils is able to chelate two ferric ions per molecules to form an iron complex. Milk lactoferrin isolated from whey is iron-unsaturated; the degree of saturation is only 25-30%. Lactoferrin releases chelated ferric ions in acidic conditions below pH 3 and becomes iron-free apolactoferrin. Thus, apolactoferrin can be utilized as a raw material to achieve the level of desired iron-saturation in lactoferrin by adding calculated amounts of ferric ions in the presence of carbonic anion. We have confirmed that the degree of iron-saturation does not affect the synergistic effect on antibacterial activities of beta-lactam antibiotics through a study using 100%, 28%, and 0% iron-saturated lactoferrin. So lactoferrins with various degrees of iron-saturation can be utilized in this invention. The synergistic effect of lactoferrin with beta-lactam antibiotics is not dependent on the degree of iron-saturation.

The stability of bovine lactoferrin powder is also not affected by the degree of iron-saturation. It is very stable at room temperature and is readily formulated into tablets, granules, tincture, and powder. Much care to avoid denaturation of lactoferrin, especially by heat, should be taken in the formulating processes. Due to its high stability, it is readily processed into mixed formulations with beta-lactam antibiotics for oral administration in any proportion, for example, freely mixing with lactose, cellulose derivatives, magnesium stearate, and talc. One of the best ways to make lactoferrin liquid medium is as follows: lactoferrin solution is adjusted to pH 3.1-4.0 by the addition of lactose, paraaminobenzoic acid added as a preservative.

The timing of oral or other administrations of lactoferrin in this invention should be either simultaneous with or shortly before or after the administration of beta-lactam antibiotics; any formulated form of lactoferrin may be used; e.g.

Lactoferrin can be utilized when mixed with foods, e.g., supplemented milk, yoghurt, skim milk powder, lactic acid bacteria fermented milk, chocolates, tablet sweets, and powdered beverages. The most important factor is to avoid protein denaturation during processing by not exceeding a temperature of about 60 C.

The pharmaceutical compositions of this invention may contain the active compounds together with a solid or liquid pharmaceutically acceptable nontoxic carrier. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solution and aqueous dextrose and glycerol solutions can also be employed as liquid carriers, particularly for injectable solutions. Suitable pharmaceutical excipients include starch, glucose, lactose, sucrose, gelatine, malt, rice, flour, chalk, silica gel, magnesium carbonate, magnesium stearate, sodium stearate, glycerol monostearate, talc, sodium chloride, dried skim milk, glycerol, propylene glycol, water, ethanol. These compositions can take the form of solutions, suspensions, tablets, pills, capsules, powders, sustained-release formulations. Suitable pharmaceutical carriers are described in "Remington's Pharmaceutical Sciences" by E. W. Martin. Such compositions will contain an effective therapeutic amount of the active compound together with a suitable amount of carrier so as to provide the form for proper administration to the host. While intravenous injection is a very effective form of administration, other modes can be employed.

### Example 1

Eighteen male I.C.R. mice (4 weeks of age, average weight 19 + 1.0 g) were randomly divided into three groups (n=6), and intraperitoneally injected with pneumobacillus, Klebsiella pneumoniae K35, 5 x 10⁶ CFU/mouse. 0.2 ml physiological saline were then orally given to the control mice 1 hour after injection of the bacteria, and 5 mg cefpodoxime in 0.2 ml physiological saline were given to the cephalosporin antibiotic control group. To the third group, the admixture of 1 mg lactoferrin and 5 mg cefpodoxime in 0.2 ml physiological saline was orally given.

The infected mice were housed in an animal room, allowed free access to food and water, and observed for a week under the controlled conditions of 22°C with 50% relative humidity.

All mice in the control group died within 24 hours, while one of six mice in the cephalosporin control group survived 1 week after infection and was regarded as completely cured from infectious disease. All the mice in the combined treatment group survived the experimental period and were regarded as completely recovered. On the basis of these results, ED₅₀'s of both cefpodoxime groups were as follows: In case of cefpodoxime alone it was 7.93 mg/mouse (6.3-10.0 mg), while in case of the combined therapy of cefpodoxime with lactoferrin it was 1.25 mg/mouse (0.92-1.7 mg). These results suggest that oral administration of lactoferrin has a remarkable synergism with the cephalosporin class of antibiotics. Without being bound by theory, it is highly unlikely that the mechanism of this synergism is related to enhancement of intestinal absorption of cefpodoxime caused by lactoferrin. The antibiotic concentrations in blood were almost the same between the two cefpodoxime groups, i.e., the cefpodoxime control group and the combined cefpodoxime-lactoferrin group. The most plausible mechanism is that lactoferrin activates the nonspecific immune system, thus killing infecting pathogens in cooperation with smaller amounts of beta-lactam antibiotics.

### Example 2

Eighteen male I.C.R. mice (5 weeks of age, average weight 19 + 1.0 g) were divided at random into three groups (n=6), and intraperitoneally injected with pneumobacilli, Klebsiella pneumoniae K35, 5 x 10⁶ CFU/mouse. 0.2 ml physiological saline were then orally given to the control mice 1 hour after injection of the bacteria, and 1 mg cefpodoxime in 0.2 ml physiological saline were orally given 1 hour after the injection to the cephalosporin control group. On the other hand, an admixture of 0.4 mg lactoferrin and 1 mg cefpodoxime in 0.2 ml physiological saline were orally given to the mice belonging to the cefpodoxime-lactoferrin group.

Mice were fed ad libitum and observed for a week under the same conditions as described in Example 1.

All mice in the control group died with 24 hours. ED₅₀'s of both cefpodoxime groups were calculated as follows: in the case of administration of cefpodoxime alone (cefpodoxime control group) it was 0.99 mg/mouse (0.74-1.32 mg), while in case of the combined treatment of cefpodoxime with lactoferrin, it was 0.25 mg/mouse (0.19-0.33).

### Example 3

Eighteen male I.C.R. mice (5 weeks of age, average weight 19 ± 1.0 g) were randomly divided into three groups (n=6), and intraperitoneally injected with pneumobacilli, Klebsiella pneumoniae K35, 5 x 10⁶ CFU/mouse. To the control mice, 0.2 ml physiological saline were orally given immediately after the injection of the bacteria, and 0.5 mg cefaclor in 0.2 ml physiological saline were orally given to the cephalosporin antibiotics control group. On the other hand, an admixture of 0.4 mg lactoferrin and 0.5 mg cefaclor in 0.2 ml physiological saline were orally given to the mice in the third group.

Mice were fed ad libitum and observed for a week under the same conditions as described in Example 1.

All mice in the control group died within 24 hours. ED₅₀'s of the cefaclor groups were calculated as follows: in the case of cefaclor alone (cephalosporin control group) it was 0.99 mg/mouse (0.74-1.32 mg), while in the case of the combined therapy of cefaclor with lactoferrin, it was 0.25 mg/mouse (0.19-0.33 mg).

### Example 4

Eighteen male I.C.R. mice (5 weeks of age, average weight 19 ± 1.0 g) were randomly divided into three groups (n=6), and intraperitoneally injected with pneumobacilli, Klebsiella pneumoniae K35, 5 x 10⁶ CFU/mouse. 0.2 ml distilled water were orally given to the control group immediately after injection of the bacteria, and 0.5 mg cefotetan in 0.2 ml distilled water were orally given to the cephalosporin antibiotic control group. On the other hand, the admixture of 0.4 mg lactoferrin and 0.5 mg cefotetan in 0.2 ml distilled water were orally given to the combined therapy group.

Mice were fed ad libitum and observed for a week under the same conditions as described in Example 1.

All the mice in the control group died within 24 hours. ED₅₀'s of the cefotetan groups calculated were as follows: in the case of the administration of cefotetan alone (cephalosporin control group) it was 0.5 mg/mouse (0.34-0.72 mg), while in the case of the combined therapy (lactoferrin treated group) it was 0.16 mg/mouse (0.11-0.22 mg).

### Example 5

Gastric emptying greatly affects intestinal absorption of some kinds of drugs, especially lipophilic ones in which absorption is greatly accelerated by the presence of foods in the stomach. Cefpodoxime is relatively lipophilic, so it was anticipated that the absorption rate is less under fasting conditions.

Twenty-four male I.C.R. mice (5 weeks of age, average weight 19 ± 1.0 g) were randomly divided into four groups (n=6). During the next 24 hours, drinking water and pellet diet were freely allowed to two groups, while the other two groups were fasted overnight. On the next day, all the mice were intraperitoneally injected with pneumobacilli, Klebsiella pneumoniae K35, 1.4 x 10⁷ CFU/mouse. The minimum lethal dose of the bacterium is 1 x 10³ CFU/mouse. 5 and 10 mg/mouse of cefpodoxime in 0.2 ml distilled water were orally given immediately after the injection of the bacteria into the two groups, the one was fed and the other fasted, respectively. To the other two fed and fasted groups, as well as the control groups, were orally given 5 and 10 mg/mouse of cefpodoxime immediately after the injection of the bacteria, accompanied with simultaneous oral administration of 0.2 mg/mouse of lactoferrin in 0.2 ml distilled water. ED₅₀'s of cefpodoxime in each group is shown in Table 2.

According to the results, antibacterial activity of cefpodoxime was elevated 5 fold when 0.2 mg/mouse of lactoferrin was simultaneously given to the infected mice, as compared with an administration of cefpodoxime alone. The synergistic effect of lactoferrin with beta-lactam antibiotics is unaffected by whether the infected animals are fasted or fed.

### Example 6

Thirty male ddy mice (5 weeks of age, average weight 21.5 g) were randomly divided into three groups (n=10), and injected with Escherichia coli #11, 1.2 x 10⁶ CFU/mouse in the abdominal cavity. To the control group, 0.2 ml distilled water were given orally immediately after the injection of the bacteria, and 2 mg ampicillin in 0.2 ml distilled water were given to the penicillin control group. On the other hand, admixture of 1 mg lactoferrin and 2 mg ampicillin in 0.2 ml distilled water were orally given to the mice in the third group.

Mice were fed ad libitum and observed for a week under the same conditions as described in Example 1.

All mice in the control group died within 24 hours. ED₅₀'s of the ampicillin groups were calculated as follows: in the case of ampicillin alone (penicillin control group) it was 6.02 mg/mouse, while in the case of the combined treatment (test group) it was 0.75 mg/mouse.

Obviously, according to the results, lactoferrin not only increases the antibacterial activity of the cephalosporin class, but also that of the penicillin class.

### Example 7

Sixty male ddy mice (5 weeks of age) were randomly divided in six groups (n=10), and each group was injected with Proteus mirabilia GN79 (1.3 x 10⁶ CFU/mouse), Escherichia coli #11 (1.7 x 10⁶ CFU/mouse), and Klebsiella pneumoniae 3K25 (1.5 x 10⁶ CFU/mouse) into the abdominal cavity. To the three control groups, three antibiotics, ampicillin, cephalexin, cefaclor, were given orally immediately after the injection of the bacteria. On the other hand, an admixture of 1 mg lactoferrin and the antibiotics were orally given to the mice in the other three groups.

Mice were fed ad libitum and observed for a week under the same conditions as described in Example 1.

As shown in Table 3, when lactoferrin was orally given simultaneously with a beta-lactam antibiotic to the mouse immediately after injection of pathogenic bacteria, antibacterial efficacy appeared to be increased 2-10 times greater than the antibiotics alone.

The above examples can be reproduced in a similar way when other routes of administration are utilized.

Further variations and modifications of the invention will become apparent to those skilled in the art from the foregoing and are intended to be encompassed by the claims appended hereto.

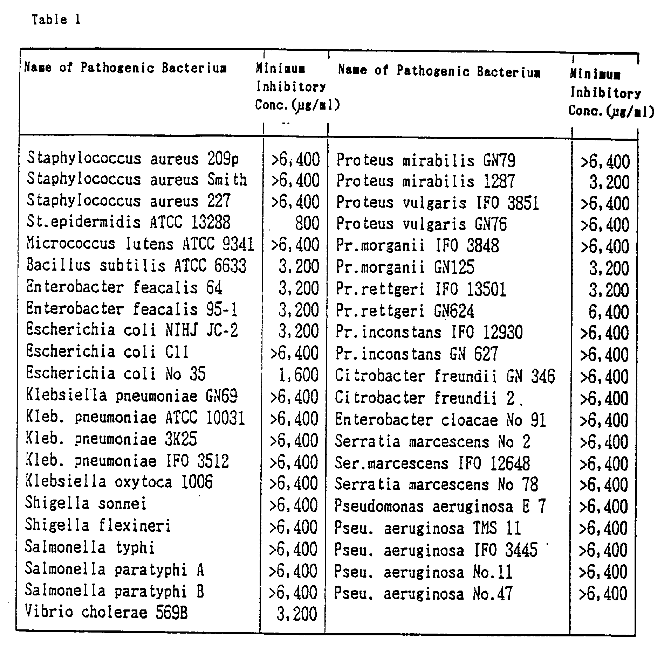

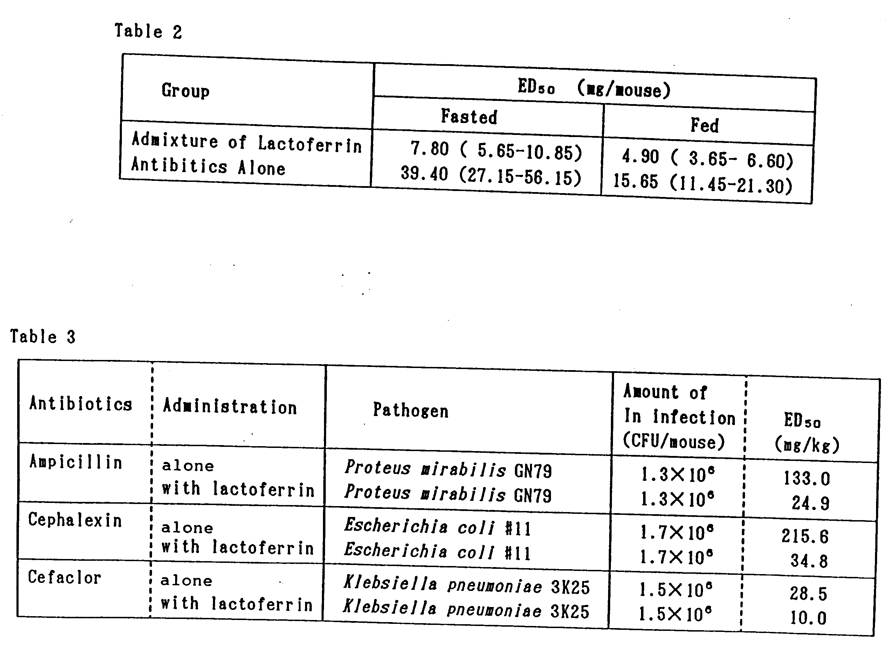

## Claims

1. A pharmaceutical composition enhancing the efficacy of beta-lactam antibiotics, comprising lactoferrin and at least one beta-lactam antibiotic.

2. The pharmaceutical composition according to claim 1, wherein said beta-lactam antibiotic is selected from the group consisting of penicillin class and cephalosporin class beta-lactam antibiotics.

3. The pharmaceutical composition according to claim 1, wherein the weight ratio of lactoferrin : beta-lactam antibiotics is 1 : 1 to 1 : 10.

4. The pharmaceutical composition according to claim 1, wherein said lactoferrin is present at a dosage of 0.5-100 mg/kg.

5. The pharmaceutical composition according to claim 4, wherein said lactoferrin is present at a dosage of 1-10 mg/kg.

6. A composition according to any of claims 1 to 5, wherein the lactoferrin is bovine lactoferrin.

7. A pharmaceutical composition enhancing the efficacy of beta-lactam antibiotics, comprising lactoferrin, at least one beta-lactam antibiotic, and a pharmaceutically acceptable carrier.

8. A food product which comprises an effective amount of a pharmaceutical composition comprising lactoferrin and optionally at least one beta-lactam antibiotic.

9. The use of lactoferrin for enhancing the activity of a beta-lactam antibiotic in the manufacture of a medicament for the treatment of infectious disease.

10. The use of lactoferrin for enhancing the activity of a beta-lactam antibiotic in the manufacture of medication for the treatment of infectious disease, the lactoferrin being formulated for administration simultaneously with or shortly before or after the administration of the beta-lactam antibiotic.

## Patentansprüche

1. Pharmazeutische Zusammensetzung zur Erhöhung der Wirksamkeit von Beta-Lactam-Antibiotica, mit Gehalt an Lactoferrin und mindestens einem Beta-Lactam-Antibioticum.

2. Pharmazeutische Zusammensetzung nach Anspruch 1, wobei das Beta-Lactam-Antibioticum aus der Penicillinklasse und/oder aus der Cephalosporinklasse ist.

3. Pharmazeutische Zusammensetzung nach Anspruch 1, wobei das Gewichtsverhältnis von Lactoferrin: Beta-Lactam-Antibioticum 1 : 1 bis 1 : 10 beträgt.

4. Pharmazeutische Zusammensetzung nach Anspruch 1, wobei das Lactoferrin in einer Dosierung von 0,5 bis 100 mg/kg enthalten ist.

5. Pharmazeutische Zusammensetzung nach Anspruch 4, wobei das Lactoferrin in einer Dosierung von 1 bis 10 mg/kg enthalten ist.

6. Zusammensetzung nach einem der Ansprüche 1 bis 5, wobei das Lactoferrin Bovinlactoferrin ist.

7. Pharmazeutische Zusammensetzung zur Erhöhung der Wirksamkeit von Beta-Lactam-Antibiotica, die Lactoferrin, mindestens ein Beta-Lactam-Antibioticum und einen pharmazeutisch verträglichen Träger enthält.

8. Nahrungsmittelprodukt mit Gehalt an wirksamer Menge einer pharmazeutischen Zusammensetzung mit Gehalt an Lactoferrin und ggfs. mindestens einem Beta-Lactam-Antibioticum.

9. Verwendung von Lactoferrin zur Erhöhung der Aktivität eines Beta-Lactam-Antibiotikums bei der Herstellung eines Medikaments zur Behandlung infektiöser Erkrankungen.

10. Verwendung von Lactoferrin zur Erhöhung der Aktivität eines Beta-Lactam-Antibiotikums bei der Herstellung eines Arzneimittels zur Behandlung infektiöser Erkrankungen, wobei das Lactoferrin zur Verabreichung gleichzeitig mit oder kurz vor oder nach der Verabreichung des Beta-Lactam-Antibioticums formuliert ist.

## Revendications

1. Composition pharmaceutique destinée à améliorer l'efficacité des antibiotiques de bêtalactamines, caractérisée en ce qu'elle comprend de la lactoferrine et au moins un antibiotique de bêtalactamines.

2. Composition pharmaceutique selon la revendication 1, caractérisée en ce que l'antibiotique de bêtalactamines est sélectionné dans la classe des pénicillines et la classe des céphalosporines de la famille des bêtalactamines.

3. Composition selon la revendication 1, caractérisée en ce que le rapport en poids de lactoferrine aux antibiotiques de bêtalactamines est de 1 à 1 jusqu'à 1 à 10.

4. Composition selon la revendication 1, caractérisée en ce que la lactoferrine est présente à un dosage de 0,5 à 100 mg/kg.

5. Composition selon la revendication 4, caractérisée en ce que la lactoferrine est présente à un dosage de 1 à 10 mg/kg.

6. Composition selon l'une quelconque des revendications 1 à 5, caractérisée en ce que la lactoferrine est une lactoferrine bovine.

7. Composition pharmaceutique pour l'amélioration de l'efficacité des antibiotiques de bêtalactamines, caractérisée en ce qu'elle présente de la lactoferrine, au moins un antibiotique de la famille des bêtalactamines et un vecteur pharmaceutique toléré.

8. Produit alimentaire caractérisé en ce qu'il comprend une quantité efficace de composition pharmaceutique comprenant de la lactoferrine et de façon optionnelle au moins un antibiotique de bêtalactamines.

9. Utilisation de lactoferrine pour améliorer l'activité d'un antibiotique de bêtalactamines pour la fabrication d'un médicament destiné au traitement d'une maladie infectieuse.

10. Utilisation de lactoferrine pour l'amélioration de l'activité d'un antibiotique de la famille des bêtalactamines dans la fabrication d'un médicament pour le traitement de maladies infectieuses, la lactoferrine étant formulée pour son administration simultanée ou juste avant ou juste après l'administration de l'antibiotique de la famille des bêtalactamines.
